Europäisches Patentamt

**European Patent Office**

**Office européen des brevets**

(19)

(11) Numéro de publication: **0 079 827**
**A1**

(12)

# DEMANDE DE BREVET EUROPEEN

(21) Numéro de dépôt: **82402057.2**

(22) Date de dépôt: **09.11.82**

(51) Int. Cl.³: **A 61 L 2/20**
**A 61 L 2/08, A 61 L 2/10**

(30) Priorité: **10.11.81 FR 8121019**

(43) Date de publication de la demande:
**25.05.83 Bulletin 83/21**

(84) Etats contractants désignés:
**AT BE CH DE GB IT LI LU NL SE**

(71) Demandeur: **TECHNO-SYNTHESE S.A. Société dite:**
**8 rue Pierre-Girard**
**F-75019 Paris(FR)**

(72) Inventeur: **Cuypers, Pascal**
**17 Villa Curial**
**F-75019 Paris(FR)**

(72) Inventeur: **Cuypers, Joseph Alphonse**
**250 Avenue Gabriel Péri**
**F-78360 Montesson(FR)**

(74) Mandataire: **Polus, Camille et al,**
**c/o Cabinet Lavoix 2, Place d'Estienne d'Orves**
**F-75441 Paris Cedex 09(FR)**

(54) Appareil pour la stérilisation de petits instruments médicaux ou para-médicaux.

(57) L'invention est relative à la stérilisation de petits instruments médicaux ou para-médicaux, tels que ceux utilisés pour l'acupuncture et la dentisterie et se propose de remédier aux inconvénients des techniques classiques en la matière. A cet effet, il est fait appel à l'ozone comme agent stérilisant à l'aide d'un appareil constitué par un coffret (1) comprenant un support (3, 3a) pour les instruments (i) à stériliser, qui délimite dans ce coffret un compartiment (5) où est disposée une source d'ozone (6), le support étant agencé de telle sorte que les parties à stériliser des instruments soient plongées dans ce compartiment.

FIG. 1

EP 0 079 827 A1

Appareil pour la stérilisation de petits
instruments médicaux ou para-médicaux.-

La présente invention est relative à la stérilisation de petits instruments utilisés à des fins médicales ou para-médicales.

Parmi ces instruments on peut notamment citer ceux utilisés :

- en dentisterie : tels que broches, vrilles, fraises, miroirs, et autres instruments de bouche;

- en acupuncture : telles qu'aiguilles;

- en mésothérapie : tels qu'aiguilles et leurs supports, et

- en médecine : tels que petits instruments de chirurgie, fibres optiques et leur optique, aiguilles pour piqûres et ponctions.

Il résulte des enquêtes effectuées auprès des milieux médicaux et dentaires, que les méthodes actuelles de stérilisation de ce type d'instruments ne donnent pas pleinement satisfaction aux utilisateurs, chacune d'elles présentant des inconvénients :

Le passage à l'étuve à haute température est certes efficace mais il est incompatible avec certains matériaux qui ne supportent pas des températures élevées ou des chocs thermiques. Par ailleurs, il est difficile de conserver la stérilité des instruments restant dans les boîtes de stérilisation, après que celles-ci aient été ouvertes. L'immersion dans un liquide bactéricide, qui est également utilisée pour la stérilisation, oblige à prendre des précautions particulières pour conserver la stérilité durant le séchage; par ailleurs, les instruments ainsi stérilisés risquent de garder des traces de produit bactéricide après séchage. Enfin, s'il a été préconisé aussi d'utiliser pour une telle stérilisation des rayons ultraviolets, ceux-ci n'agissent que par rayonnement direct et n'ont pas d'action sur les surfaces non irradiées ou dans les anfractuosités.

Le besoin se fait sentir d'une nouvelle technique de stérilisation dans ce domaine et l'invention a ainsi pour objet un appareil de stérilisation pour ce type d'instruments faisant appel comme agent stérilisant à l'ozone, déjà utilisée pour la stérilisation dans d'autres domaines.

L'appareil de stérilisation suivant l'invention comporte un coffret muni d'une cloison qui sépare de l'atmosphère extérieure un compartiment où est disposée une source d'ozone; adapté à la stérilisation de petits appareils médicaux ou para-médicaux, il est caractérisé en ce que ladite cloison aménage un support coplanaire pour les instruments à stériliser agencé de telle sorte que les parties à stériliser des instruments soient plongées dans ce compartiment où s'exerce essentiellement l'action de l'ozone. Ce support peut ainsi être formé par un plateau perforé formant la paroi supérieure du compartiment du coffret et dans les perforations duquel sont enfilés les instruments dont la partie pénétrant à l'intérieur dudit compartiment est soumise à l'action stérilisante de l'ozone.

Selon une autre caractéristique, le compartiment de stérilisation peut être délimité supérieurement par une cloison pourvue de larges ouvertures susceptibles d'être obturées par des éléments de support amovibles des instruments.

La source d'ozone est une lampe et avantageusement une lampe froide.

D'autres caractéristiques et avantages de l'invention ressortiront de la description détaillée qui va suivre de certains de ces modes de réalisation. On se référera pour cela au dessin annexé donné uniquement à titre d'exemple et sur lequel :

- la Fig. 1 est une vue en perspective d'un premier mode de réalisation de l'invention;

- la Fig. 2 est une vue en perspective d'une variante de support utilisable dans l'appareil selon l'invention, et

- la Fig. 3 est un schéma électrique illustrant la source d'ozone utilisable dans l'appareil selon l'invention.

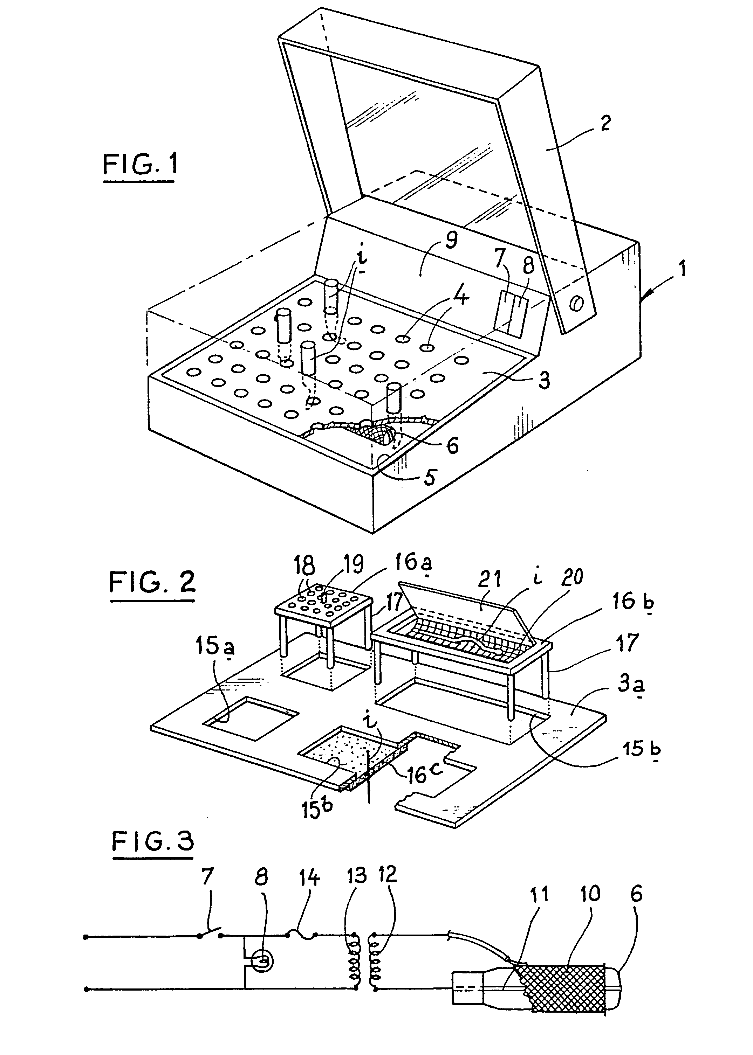

Selon le mode de réalisation représenté à la Fig. 1, l'appareil suivant l'invention comprend un coffret 1 en toute matière appropriée, telle qu'une matière plastique, pourvu d'un couvercle articulé 2 avantageusement transparent. Un plateau 3, pourvu d'une série de perforations 4, forme la paroi supérieure d'un compartiment 5 ménagé dans le coffret et dans lequel est logée une source d'ozone 6, constituée par une lampe ainsi qu'on le verra ultérieurement.

Le plateau 3 est incliné en formant ainsi un présentoir pour les instruments (i) à stériliser. Ceux-ci sont enfilés dans les perforations 4 du plateau 3 de manière que leurs parties à stériliser pénètrent dans le compartiment 5, où ils sont soumis à l'action de la source d'ozone 6, seuls les manches des instruments faisant saillie au-dessus du plateau 3. La source d'ozone 6 est commandée par un interrupteur 7 avec voyant de contrôle 8, l'ensemble étant monté dans un rebord 9 du coffret sur lequel s'articule le couvercle 2.

La lampe 6 peut être constituée d'une enveloppe en quartz pourvue d'un filament alimenté sous 24 v et 50 Hz et contenant une petite quantité de mercure. Le chauffage du filament provoque la vaporisation du mercure et les effluves engendrent des rayons ultraviolets, notamment suivant la longueur d'onde de 1850 Angströms qui est la plus propice à la production d'ozone lorsqu'elle frappe les molécules d'oxygène.

4

Le quartz est utilisé pour l'enveloppe car c'est le seul verre perméable à cette longueur d'onde. L'avantage d'une telle lampe est son alimentation sous une basse tension, souhaitable du point de vue de la sécurité. Par contre, cette lampe provoque un dégagement de chaleur non négligeable, susceptible de provoquer un ressuage du plastifiant contenu dans les matières plastiques utilisées pour la fabrication du coffret 1. Le plastifiant peut alors se transformer au contact de l'ozone en un produit visqueux d'aspect brunâtre susceptible de se déposer sur les parois du compartiment 5 et aussi sur les instruments stérilisés.

C'est pourquoi l'on donne la préférence à l'utilisation de lampes froides pour la production de l'ozone. C'est une telle lampe et son montage qui sont illustrés sur la figure 3. Cette lampe 6 est constituée d'une enveloppe en verre ordinaire entourée d'une grille métallique 10 et contenant en son sein une électrode métallique 11. Cette lampe est terminée par un culot standard soit du type à baïonnette soit du type à vis. L'alimentation se fait sous une tension comprise entre 2000 et 3000 v sous 50 Hz. Cette tension est assurée par le secondaire 12 d'un transformateur dont le primaire 13 est branché sur le secteur. Le circuit primaire comporte, outre l'interrupteur 7 et le voyant 8, un -fusible 14.

L'effluvage interne de la lampe 6 sous la tension de 2000 à 3000 v produit des ions dont un grand nombre traverse les vides de l'électrode externe 10 et qui, en frappant les molécules d'oxygène produisent de l'ozone, sans provoquer d'élévation notable de la température. Malgré la tension élevée, la sécurité de l'appareil peut être assurée sans encombre par les techniques de double isolation; par ailleurs, les courants mis en jeu de l'ordre de quelques microampères sont nettement

5

inférieurs aux valeurs admises comme dangereuses.

Le support des instruments à stériliser peut avoir une forme différente de celle illustrée à la figure 1. C'est ainsi qu'une variante a été illustrée sur la figure 2. Cette variante comporte, en place du plateau perforé 3, une cloison 3a délimitant supérieurement le compartiment 5 et pourvu d'ouvertures 15a ou 15b. Les ouvertures 15a ont été représentées de section carrée et les ouvertures 15b de section rectangulaire. Il va de soi, cependant, que la cloison 3a peut comprendre uniquement des ouvertures carrées 15a, ou uniquement des ouvertures rectangulaires 15b ou, comme représenté, une combinaison d'ouvertures 15a et 15b. Ces ouvertures sont destinées à être obturées par des éléments de support des instruments à stériliser 16a, 16b et 16c respectivement. Les éléments de support 16a et 16b sont amovibles et constitués de tablettes munies de pieds 17, susceptibles de reposer sur le fond du compartiment 5. Le plateau des tablettes 16a est une simple plaque perforée dont les orifices 18 jouent le rôle des orifices 4 du plateau 3. Cependant, la tablette 16a présente un avantage supplémentaire du fait de son amovibilité : elle peut servir à recevoir les instruments après utilisation pour les passer tels quels dans une machine à laver, par exemple à ultrasons, et être ensuite replacée dans l'appareil de stérilisation sans manipulation ou rangement supplémentaire. Pour faciliter la manipulation des tablettes 16a, un bouton de préhension 19 est prévu au centre de sa surface supérieure.

Le plateau des tablettes 16b est formé par un grillage 20 qui permet d'y disposer tous les instruments qui ne sont pas susceptibles d'entrer dans des perforations telles que 4 ou 18. Un couvercle articulé 21 permet d'étancher le berceau formé par le grillage 20. Mis à part le fait que les tablettes 16b permettent de loger une plus grande variété d'instruments que les

6

tablettes 16a, elles peuvent être utilisées exactement de la même manière que ces dernières.

L'élément de support 16c est un tampon en une matière aisément perforable, telle qu'un tissu ou mieux un non-tissé (feutre) en fibres résistant à l'ozone. Il est ainsi possible de planter dans ce tampon une ou plusieurs aiguilles lisses i d'acupuncture de manière que la partie active de celles-ci traverse le tampon et pénètre dans le compartiment 5 contenant l'ozone. Le tampon 16c a une épaisseur appropriée - de 1,5 à 3 mm par exemple - pour, d'une part, maintenir les aiguilles droites et, d'autre part, augmenter la tenue mécanique. Le tampon peut être fixé à la cloison 3a de toute manière voulue, par exemple comme représenté par collage sur la face inférieure de cette cloison le long du bord de l'ouverture 15b qu'il obture.

Quel que soit le mode de réalisation adopté, on voit que l'appareil suivant l'invention permet la stérilisation efficace de petits instruments médicaux ou para-médicaux par leur immersion dans un compartiment rempli d'ozone, ce compartiment étant obturé de manière plus ou moins étanche par le support même des instruments, l'étanchéité obtenue étant en tout état de cause suffisante pour les besoins pratiques. Cet appareil, d'un maniement simple et commode, est, par ailleurs, dépourvu des inconvénients résultant de l'emploi des techniques antérieurement utilisées aux mêmes fins.

7

## REVENDICATIONS

1 - Appareil de stérilisation comportant un coffret muni d'une cloison qui sépare de l'atmosphère extérieure un compartiment où est disposée une source d'ozone, cet appareil, adapté à la stérilisation de petits appareils médicaux ou para-médicaux, étant caractérisé en ce que ladite cloison aménage un support coplanaire (3, 3a) pour les instruments à stériliser (i) agencé de telle sorte que les parties à stériliser des instruments soient plongées dans ce compartiment (5) où s'exerce essentiellement l'action de l'ozone.

2 - Appareil suivant la revendication 1, caractérisé en ce que la cloison est formée par un plateau perforé (3), dans les perforations (4) duquel sont enfilés les instruments (i) pour que la partie pénétrant à l'intérieur dudit compartiment (5) soit soumise à l'action stérilisante de l'ozone.

3 - Appareil suivant la revendication 1, caractérisé en ce que la cloison (3a) est pourvue de larges ouvertures (15a, 15b) susceptibles d'être obturées par des éléments du support (16a, 16b, 16c) des instruments.

4 - Appareil suivant la revendication 3, caractérisé en ce que les éléments de support sont amovibles et constitués par des tablettes (16a, 16b), pourvues de pieds (17) destinés à reposer sur le fond du compartiment (5) et de plateaux constitués soit par une plaque munie d'orifices (18), soit par un berceau grillagé (20) muni d'un couvercle d'obturation (21).

5 - Appareil suivant la revendication 3, caractérisé en ce que les éléments de support sont des tampons en une matière aisément perforable et ainsi susceptibles d'être traversés par des instruments, telles des aiguilles lisses d'acupuncture, qui y sont plantées.

6 - Appareil suivant l'une quelconque des revendications précédentes, caractérisé en ce que la

8

source d'ozone est une lampe froide alimentée sous
haute tension et comportant une électrode externe
en forme de grille métallique.

0079827

1/1

FIG.1

FIG. 2

FIG.3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | A 61 L    2/20 |
| X | FR-A-2 351 666  (GREZE ANDRE) <br> * en entier * | 1-3 | A 61 L    2/08 <br> A 61 L    2/10 |
| | --- | | |
| X | DE-A-1 953 418  (KRALOVOPOLSKA STROJIRNA, NARODNI PODNIK) <br> * en entier * | 6 | |
| | --- | | |
| Y | US-A-3 114 038  (M.S. MEADER) <br> * figure 1; colonne 3, ligne 3 - colonne 6, ligne 57 * | 1-4 | |
| | --- | | |
| Y | DE-A-2 904 391  (MÜRLE, R.) <br> * page 6, alinéa 4 - page 9, alinéa 2; figures * | 1-4 | |
| | --- | | **DOMAINES TECHNIQUES RECHERCHES (Int. Cl. ³)** |
| Y | FR-A-2 251 520  (SOCIETE D'ETUDES ET DE RECHERCHES POUR LA MISE AU POINT D'APPAREILS ELECTRONIQUES ET ELECTRIQUES S.E.R.M.A.E.E.) <br> * page 1, ligne 28 - page 2, ligne 39; figure 2 * | 1,6 | A 61 L |
| | --- | | |
| Y | DE-A-3 006 487  (SCHUBERT, S.) <br> * page 4, alinéa 2 - page 6, alinéa 1; figures * | 1,6 | |
| | --- | | |
| A | US-A-3 748 094  (JOHN P. SCHEIDELL) <br> * colonne 1, ligne 49 - colonne 2, ligne 14; figures * | 1-3 | |
| | ---            -/- | | |

Le présent rapport de recherche a été établi pour toutes les revendications

| Lieu de la recherche <br> LA HAYE | Date d'achèvement de la recherche <br> 17-01-1983 | Examinateur <br> SCHRIJVERS H.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82

## DOCUMENTS CONSIDERES COMME PERTINENTS

Page  2

| Catégorie | Citation du document avec indication, en cas de besoin, des parties pertinentes | Revendication concernée | CLASSEMENT DE LA DEMANDE (Int. Cl. ³) |
|---|---|---|---|
| A | US-A-2 264 313  (A.A. HUMPHREY) <br> * colonne  2, ligne 35 - colonne 3, ligne 59 * <br><br> --- | 1,4,5 | |
| A | US-A-2 775 005  (MILTON B. WEINBERGER) <br> * en entier * <br><br> ----- | 1 | |
| | | | **DOMAINES TECHNIQUES RECHERCHES (Int. Cl. ³)** |

Le présent rapport de recherche a été établi pour toutes les revendications

| Lieu de la recherche <br> LA HAYE | Date d'achèvement de la recherche <br> 17-01-1983 | Examinateur <br> SCHRIJVERS H.J. |
|---|---|---|